# EUROPEAN PATENT SPECIFICATION

(11) **EP 4 382 036 B1**
(45) Date of publication and mention of the grant of the patent: **02.09.2026**
(21) Application number: 23211607.9
(22) Date of filing: 22.11.2023
(51) Int. Cl.: A61B 5/00, A61B 5/055, A61B 5/11, A61B 6/00, G01R 33/565

(54) **BODY MOVEMENT INFORMATION PROCESSING DEVICE AND MAGNETIC RESONANCE IMAGING DEVICE**
VORRICHTUNG ZUR VERARBEITUNG VON KÖRPERBEWEGUNGSINFORMATIONEN UND MAGNETRESONANZBILDGEBUNGSVORRICHTUNG
DISPOSITIF DE TRAITEMENT D'INFORMATIONS DE MOUVEMENT CORPOREL ET DISPOSITIF D'IMAGERIE PAR RÉSONANCE MAGNÉTIQUE

(30) Priority: 07.12.2022 JP 2022195950
(43) Date of publication of application: 12.06.2024
(73) Proprietor: FUJIFILM Corporation, Tokyo 106-8620 (JP)
(72) Inventor: KANEKO, Yukio, Chiba (JP); SHIRAI, Toru, Chiba (JP); AMEMIYA, Tomoki, Chiba (JP); NAGAO, Hisako, Chiba (JP); IKEGAWA, Ayaka, Chiba (JP); SAKURAGI, Kenta, Chiba (JP); MURASE, Takenori, Chiba (JP); SHOJI, Hiroki, Chiba (JP)
(74) Representative: Strehl & Partner mbB

(56) References cited:
- EP-A1- 3 912 556
- EP-A2- 3 639 738
- WO-A1-2016/030537
- US-A1- 2014 378 816
- US-A1- 2022 202 376

## Description

The present application claims priority from Japanese patent application JP-2022-195950 filed on December 7, 2022.

### BACKGROUND OF THE INVENTION

### 1. Field of the Invention

The present invention relates to a device that processes information regarding a subject's movement during an examination using an image diagnostic device, and particularly, to a technology for monitoring a sudden or non-steady movement during the examination.

### 2. Description of the Related Art

In an examination using an image diagnostic device such as a magnetic resonance imaging device (hereinafter, referred to as an MRI device), a subject (patient) undergoes the examination in a lying-down state on a table or the like or in a gently secured state. In a case where the subject moves during this examination, an artifact is generated in an image obtained by the image diagnostic device, which may hinder the diagnosis. In addition, in a case where the subject's movement is significant and sudden, the subject deviates significantly from an examination position, or the subject falls from a bed, the examination should be interrupted, but during the examination, a technician or a doctor (referred to as a user) often has their attention focused on a monitor displaying the image and may not notice such incidents.

Conventionally, as technologies related to the image diagnostic device, a technology for reducing an influence of the subject's movement during the examination on a diagnostic image has been developed and proposed.

For example, various technologies have been proposed such as a technique of understanding in advance a period or a phase of body movements for periodic movements including respiratory movements and heart rate movements among the subject's movements and performing body movement-synchronized imaging based on the period or the phase, or a technique for correcting data obtained after imaging by using body movement information, and suppression of artifacts caused by the body movements has been realized.

As a technology applicable to non-periodic movements, for example, JP2006-346235A has proposed an MRI device in which a body movement is detected by a camera attached within or near an examination space and scanning is stopped or performed again in a case where the body movement is detected. In addition, a technology for estimating the subject's movement from data itself acquired by the image diagnostic device has also been proposed. In a technology described in JP2021-29777A, magnetic resonance data is collected through multi-shots, and presence or absence of the body movement is estimated from low-frequency region data of each shot and used for correction.

Additionally, although it is not a technology related to an image diagnosis, R. Janssen, et al. "Video-based respiration monitoring with automatic region of interest detection", Physiological Measurement, 37 (1), 100 to 114 discloses a technology for monitoring respiratory movements by using a video image.

A device from which the pre-characterizing part of claim 1 starts out is disclosed in US 2022/202376 A1.

### SUMMARY OF THE INVENTION

In the technology disclosed in JP2006-346235A, a correlation between a still image and a time-phase image acquired at a predetermined time interval is automatically determined, and scanning is stopped or rescanning is performed according to the determination, which cannot specify a position of the subject causing the movement. Therefore, there is a probability of scanning being stopped even in a case where it is not necessary to stop the scanning, for example, in a case where there is a movement in a leg part during imaging of a head part. In addition, since the nature of the movement is not determined or displayed, there is also a probability of an examination technician or a doctor going unnoticed even in a case where there is a significant change in movement that should lead to the interruption of the examination.

In the technology described in R. Janssen, et al. "Video-based respiration monitoring with automatic region of interest detection", Physiological Measurement, 37 (1), 100 to 114, respiratory movements are separated from noise by determining an ROI of the respiratory movements, and respiratory movement signals are extracted, but movements other than respiratory movements are not considered as a target, and even in a case where this technology is applied to the examination using the image diagnostic device, the effectiveness is limited.

Since, in the technique described in JP2021-29777A, the determination is made based on the signals from an examination part, there is no concern about performing unnecessary processing for the movement of a part that does not affect the examination, but the technique is based on a multi-shot multi-echo sequence as a premise and has limitations in imaging sequences.

An object of the present invention is to provide a technology for enabling accurate understanding of a movement of a region that affects imaging, particularly, processing corresponding to a sudden or non-steady movement, and another object is to reduce an influence of the movement without limitations on an imaging method.

In order to achieve the above objects, the present invention relates to acquiring body movement information based on a signal from a measuring device that measures a movement of a subject, determining a region where the movement of the subject has occurred, calculating a body movement for this region, and determining a non-steady movement.

That is, according to an aspect of the present invention, there is provided a body movement information processing device as defined by the appended claims.

The magnetic resonance imaging device of the aspect of the present invention also includes a magnetic resonance imaging device including the measuring device as an accessory device.

Furthermore, according to still another aspect of the disclosure, there is provided a body movement information processing method.

According to the aspects of the present invention, by determining the region where the movement of the subject has occurred and extracting the body movement for that region, it is possible to appropriately take measures such as interruption of the examination or re-imaging. By appropriately setting the region, it is possible to present the respiratory movement and other movements by discriminating between the respiratory movement and other movements. Further, according to the aspects of the present invention, the body movement information is acquired by processing the signal from the measuring device, so that the present invention can be applied to any imaging technique.

### BRIEF DESCRIPTION OF THE DRAWINGS

Fig. 1 is a diagram showing an outline of an imaging system according to the present invention.
Fig. 2 is a diagram showing an overall outline of an MRI device as an example of an imaging device.
Fig. 3 is a diagram showing a relationship between a biological information measuring device and an imaging device.
Fig. 4 is a diagram showing a processing flow of a body movement information processing device of Embodiment 1.
Fig. 5 is a functional block diagram of the body movement information processing device of Embodiment 2.
Fig. 6 is a diagram showing a processing flow of the body movement information processing device of Embodiment 2.
Fig. 7 is a diagram illustrating a mask.
Fig. 8 is a diagram illustrating weighting of the mask.
Fig. 9 is a diagram showing a concept of a three-dimensional mask.
Fig. 10 is a diagram showing an example of body movement information obtained by the body movement information processing device.
Fig. 11 is a diagram showing a display example of an alert.
Fig. 12 is a diagram showing a processing flow of the body movement information processing device of Embodiment 2.
Fig. 13 is a functional block diagram showing an embodiment of the MRI device.

### DESCRIPTION OF THE PREFERRED EMBODIMENTS

Hereinafter, embodiments of an imaging system and a body movement information processing device according to the present invention will be described.

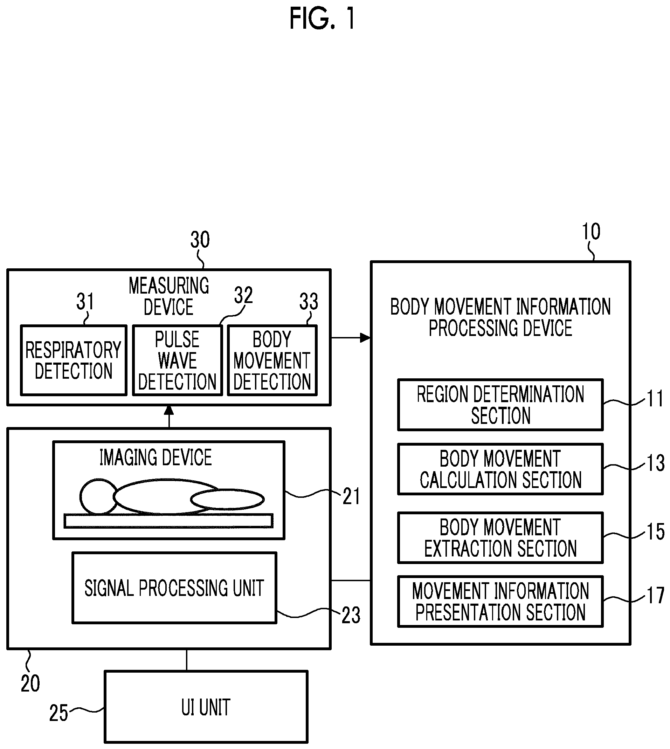

As shown in Fig. 1, an imaging system 1 comprises a medical imaging device (simply referred to as an imaging device) 20 that acquires a diagnostic image of a subject, a biological information measuring device (simply referred to as a measuring device) 30 that measures biological information of the subject disposed in an examination space of the imaging device 20, and a body movement information processing device 10 that collects information regarding a movement of the subject by using the biological information output by the measuring device 30.

The medical imaging device 20 is an image diagnostic device such as an MRI device, a CT device, an X-ray diagnostic device, or a PET, comprises the examination space in which the subject is placed, and comprises an imaging unit 21 that performs, for example, operations such as irradiation and reception of high frequency magnetic fields, X-ray irradiation, and detection of transmitted X-rays in order to generate an image of the subject, and a signal processing unit 23 that generates an image such as a tomographic image by using a signal collected by the imaging unit 21 or performs a control or the like necessary for the operations of the imaging unit 21. In addition, a UI unit 25 that includes a display device for displaying the generated image or displaying a GUI for receiving a command from a user (an examination technician or a doctor) is provided.

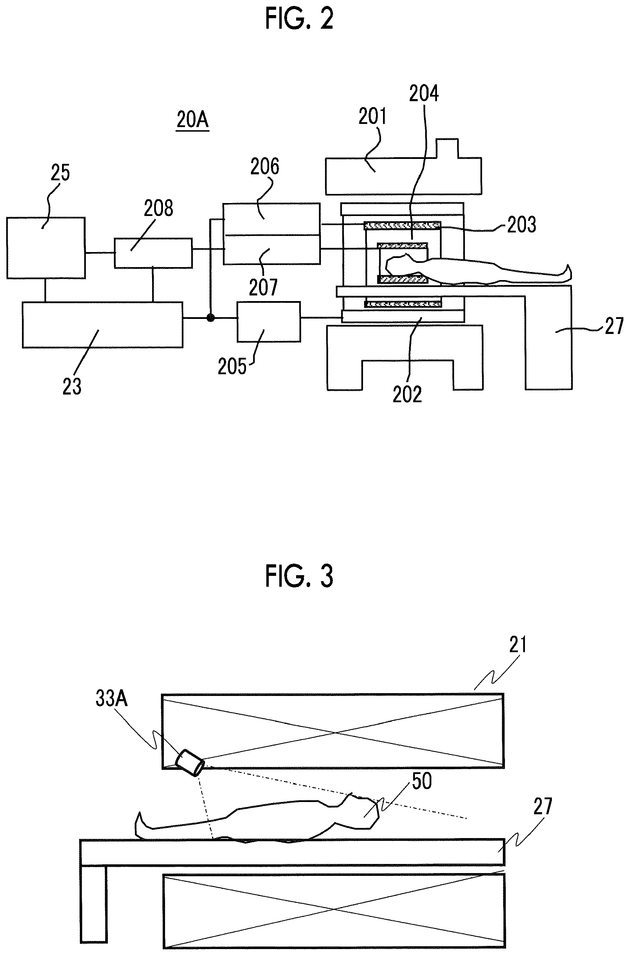

In the present embodiment, as an example, a case where the medical imaging device 20 is an MRI device will be described. An MRI device 20A has the same configuration as that of a general MRI device, and as shown in Fig. 2, comprises, as the imaging unit 21, a magnet 201 for generating a static magnetic field in the examination space, a gradient magnetic field coil 202 and its driving power supply 205 for applying a gradient to the static magnetic field (gradient magnetic field generation system), an RF transmission coil 203 and a transmitter 206 for irradiating the subject with high frequency magnetic fields (transmission system), an RF receive coil 204 and a receiver 207 for receiving a magnetic resonance signal from the subject (reception system), a sequencer (not shown) for controlling the gradient magnetic field generation system, the transmission system, and the reception system, and the like. The subject is transported to the examination space, which is a static magnetic field space, in a state of being placed on a bed 27 and undergoes the examination.

In the MRI device, k-space data necessary for image reconstruction is collected by operating the gradient magnetic field generation system, the transmission system, and the reception system in accordance with a predetermined pulse sequence. The signal processing unit 23 performs image reconstruction operations such as Fourier transformation and sequential reconstruction processing on the k-space data collected by the reception system to generate an MR image. In addition, the signal processing unit 23 performs body movement correction by using the body movement information sent from the body movement information processing device 10, which will be described below, or sends a command to the imaging unit 21 as necessary to stop imaging, perform re-imaging, or the like. The MR image generated by the signal processing unit 23, a GUI for the user to input a command, and the like are displayed on a display device of a console (UI unit) 25 provided with a display device or an input device.

The measuring device 30 consists of one or a plurality of devices that use direct or indirect means to collect the biological information such as respirations, heart rate, and body movements of the subject disposed in the examination space of the imaging device 20, and in the shown example, a respiration monitor 31 such as a balloon attached to the subject, a heart rate monitor 32 such as an electrocardiogram or a heart rate meter, and a body movement monitor 33 are shown. However, in the present invention, the respiration monitor and the heart rate monitor are not essential. The body movement monitor 33 is a device for detecting movements of the subject including respiratory movements, particularly non-steady movements, sudden movements, and the like that are difficult to capture with the respiration monitor or the heart rate monitor, and for example, a camera (including a stereo camera, an infrared camera, or the like) can be used. In the following description, a case where the body movement monitor 33 is a camera will be described as an example.

Fig. 3 shows an example in which the camera is installed in the examination space of the imaging device 20. In this example, the imaging device 20 is an MRI device, a table on which a subject 50 is placed is inserted into a bore (examination space) of the MRI device, and a camera 33A is installed near an insertion-side end part of the bore and on an upper surface and is installed to display substantially the entire body of the subject placed on the table. In a case of installing two cameras or in a case of using a stereo camera, the camera can be disposed at a predetermined angle in a direction orthogonal to a major axis direction of the bore.

The biological information measured by the measuring device 30 is presented to the user via a display unit provided in each monitor or the display device of the console 25. The video of the camera 33A is sent to the body movement information processing device 10.

The body movement information processing device 10 uses the video from the camera 33A to detect the movement of the subject, calculate the body movement (a displacement amount and a direction), and present information regarding the time or amount of the body movement that has occurred to the user or transfers this information to the imaging device 20. In this case, a region to be monitored is determined, and information about the determined region is provided. The region to be monitored may be, for example, a region that includes an imaging target part of the subject, or a relatively wide region that includes the imaging target part and other regions. For example, in order to realize a function of performing body movement correction using the body movement information on the image generated by the imaging device 20 (hereinafter, referred to as a body movement correction function), the region including the imaging target part of the subject need only be monitored, and it is preferable to monitor the relatively wide region in order to realize a function of watching a state of the subject, such as in a case where the subject has convulsions or falls from the table (hereinafter, referred to as a watching function).

In order to perform these functions, the body movement information processing device 10 of the present embodiment comprises a region determination section 11, a body movement calculation section 13, a body movement extraction section 15, and a movement information presentation section 17. The body movement information processing device 10 can be configured by, for example, a computer provided with a memory and a CPU/GPU, and a function of each unit of the body movement information processing device 10 can be realized by the CPU or the like through the reading of a program for realizing this function. In addition, some of the functions may be realized by a programmable IC such as ASIC or FPGA. Note that Fig. 1 shows a case where the body movement information processing device 10 is a device separated from the imaging device 20, but the signal processing unit 23 of the imaging device 20 can be provided with the function of the body movement information processing device 10, and such a configuration is also included in the imaging system of the embodiment of the present invention.

### Embodiment 1

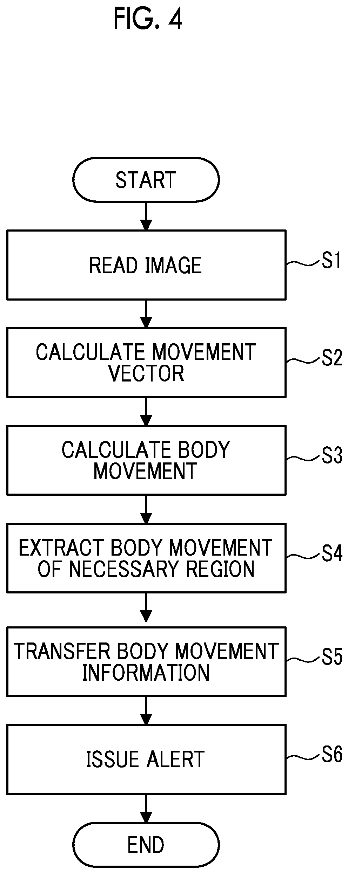

Hereinafter, an embodiment of processing of the body movement information processing device 10 will be described using a case where the imaging device 20 is an MRI device as an example. An outline of the processing is shown in Fig. 4.

First, in a case where an image is read from the camera 33A (S1), the body movement calculation section 13 calculates a movement vector (S2). The movement vector can be calculated using, for example, a known computer vision technology such as optical flow, and the movement vector is calculated for each pixel of the image.

Next, the body movement is calculated using the movement vector (S3). In a case where the camera 33A is a stereo camera, the body movement is obtained as an absolute value because the distance from the camera is obtained, but in a case of one camera, the body movement is calculated as a relative value. The body movement is calculated as a value for each small region obtained by dividing the image into a plurality of regions. In addition, in the calculation of the body movement, in order to distinguish between respiratory movements in which vertical movements of the abdomen are predominant and non-periodic body movements (also simply referred to as a body movement) in which the direction of the movement is not regular, different calculation equations may be used. Details of the calculation technique will be described in the embodiment to be described below.

The body movement extraction section 15 extracts a region that should be monitored by using a mask for extracting the region that should be monitored for body movements (S4). The region determination section 11 determines the region that should be monitored for body movements and sets a mask. The region that should be monitored for body movements differs depending on whether it is the body movement correction function or the watching function, and either one may be used exclusively, but both functions can also be concurrently performed. In such a case, the region determination section 11 determines at least two regions.

The movement information presentation section 17 determines, regarding the body movement of a predetermined region extracted by the body movement extraction section 15, whether the body movement correction is necessary or an alert needs to be issued by using a threshold value of a magnitude or a duration time of the body movement, and sends the obtained body movement information to the imaging device 20 (S5) and presents the obtained body movement information as the alert as necessary (S6).

The body movement information processing device 10 continuously executes the above-described processing S1 to S6 for the video continuously sent from the camera 33A, and provides the body movement information to the imaging device and the user who operates the imaging device.

According to the present embodiment, by determining the region that should be monitored and monitoring the body movement of that region, it is possible to avoid a problem of over-detection such as stopping imaging due to movements of a part that does not affect the imaging. In addition, since an alert is issued even in a case where there is a significant movement that should lead to the interruption of the examination in a part other than the part to be imaged, this alerts the user and allows for a safe examination. Further, according to the present embodiment, since the movement of the subject is detected and processed using only the information from the biological information measuring device independent of the imaging technique of the imaging device, the body movement correction or the watching of the subject can be realized without imposing limitations on the imaging method.

Hereinafter, a more detailed embodiment of the function of the body movement information processing device will be described.

### Embodiment 2

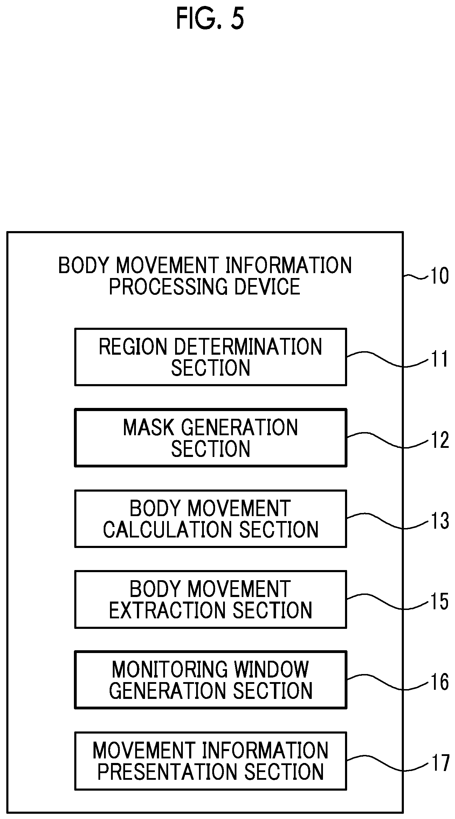

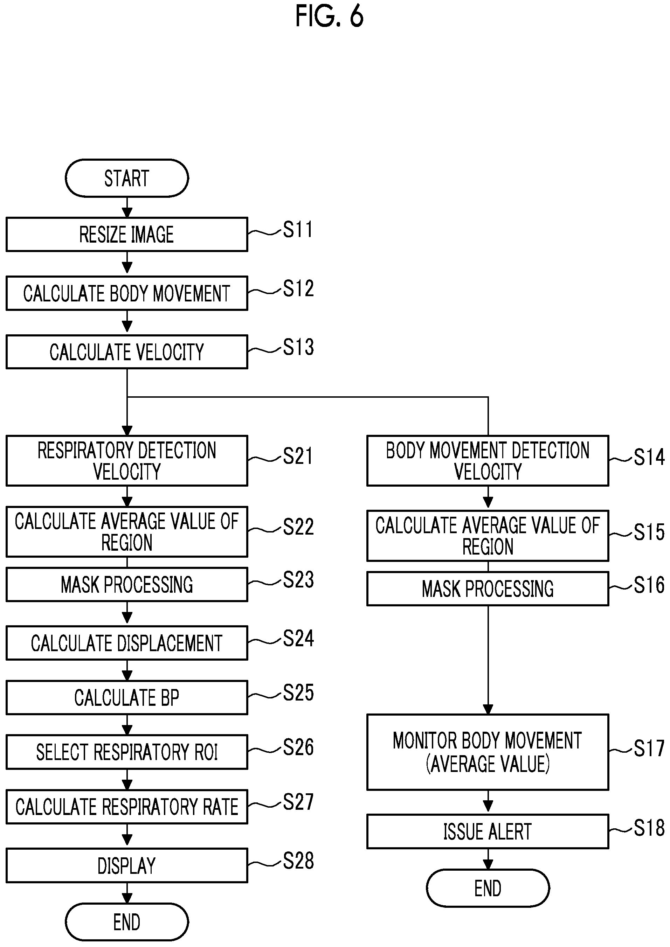

In the present embodiment, a case where both the body movement correction function and the watching function are realized, that is, a case where the respiratory movements and other non-steady body movements are processed will be described. Fig. 5 shows a functional block diagram of the body movement information processing device 10 that realizes the functions of the present embodiment. In Fig. 5, the same elements as elements shown in Fig. 1 are indicated with the same reference numerals. The functions of the elements shown in Fig. 5 will be described together with a processing flow of the present embodiment. The processing flow is shown in Fig. 6, but the flow of Fig. 4 used in Embodiment 1 is referred to as necessary.

### Movement Vector Calculation S2

The body movement calculation section 13 reads a camera image. The image is resized and the original image size is downsized, as necessary (S11). For example, the original size of the camera image is reduced to about 1/2 to 1/10. By reducing the size of the image in this way, it is possible to reduce the computational load for subsequent processing such as the movement vector calculation, which allows for computational acceleration (real-time presentation). Further, in the succeeding processing, in order to divide one image into a plurality of regions having the same pixel size, image end parts of the resized image, which are remainders, are cut such that both the height and the width are divisible by the number of divisions.

Next, the movement of each pixel is calculated using a computer vision technology such as optical flow (S12). As an optical flow algorithm, for example, a Ferneback method, a Lucas-Kanade method, and the like are known, and any of these methods may be employed. The calculated result represents a change in the pixel positions between frames for each pixel as a velocity vector, and in a case where the height and the width of the image are denoted by an x direction and a y direction, these changes are calculated as a velocity in the x direction (Vx) and a velocity in the y direction (Vy), respectively (S13).

### Body Movement Calculation S3

The body movements are calculated using the following equations using the velocity vectors Vx and Vy (S21, S14). **In** this case, calculations using different calculation equations are performed by taking into consideration the difference in the characteristics between both the movements. The respiratory movements are mainly characterized by predominant vertical movements with respect to a horizontal plane (a table surface) in a case where the subject is in a lying posture on the table, whereas the body movements exhibit movements in irregular directions. **In** a case where the main direction of the respiratory movement is the y direction, and the respiratory movement is denoted by Vresp, Vresp is represented by Equation (1). $Vresp = Vy$

The body movement is denoted by Vbody and is set as follows, for example. $Vbody = Vx$

Vbody can take positive and negative values depending on the direction of the body movement. However, in a case where it is desired to obtain the magnitude of the body movement as an absolute value, for example, Equation (2) may be calculated. $Abody = {\left(Vx\right)}^{2}$

Depending on the attachment angle of the camera, the main direction of the respiratory movement may be different from Vx or Vy. In that case, for example, Vresp is calculated by using Equation (3) using θ. $Vresp = Vx cosθ - Vy sinθ$

For example, in a case where it is desired to detect other body movements such that the respiratory movement is not included as much as possible by considering that the direction of the body movement is random, the other movements are calculated using Equation (4) by taking into consideration the movement in a direction orthogonal to Vresp. $Vbody = Vx sinθ + Vy cosθ$

In a case where it is desired to obtain the magnitude of the body movement as an absolute value, the magnitude is calculated as Abody by using, for example, Equation (5) or Equation (6). $Abody = {\left(Vbody\right)}^{2}$ $Abody = {\left(Vx sinθ\right)}^{2} + {\left(Vy cosθ\right)}^{2}$

In the equation, the method of determining θ differs depending on, for example, a positional relationship with the camera, which is an angle of an XY plane with respect to the horizontal plane, and the like. In addition, θ may be determined after analyzing the direction of the respiratory movement for each patient. θ can be appropriately set such that the respiratory movements and the body movements can be detected with high sensitivity, and different values may be used for each position (pixel).

The resized image is divided into small regions, and average values of velocity vectors Vx2 and body movement vectors Ay2 obtained for each pixel within the small region are calculated for each of the divided small regions (S22, S15). As an example, in a case where the pixel size of the camera image, after resizing and removing the remainders, is 360 in width × 270 in height, the size of one small region obtained by dividing the camera image into 60 regions is 36 × 45 pixels, and the average values of the velocity vectors and the body movement vectors are calculated for each small region having such a size.

### Body Movement Extraction (Mask Processing) S23, S16

After the calculation of the body movement, the region determination section 11 determines the region that should be monitored, and the body movement of the region is extracted.

The determination of the region that should be monitored is performed according to, for example, whether it is a region centered on the imaging range (mainly monitoring for the body movement correction function) or a region including the imaging range and other ranges (mainly monitoring for the watching function), and a predetermined region is extracted using a mask. The mask is generated in advance by, for example, a mask generation section 12.

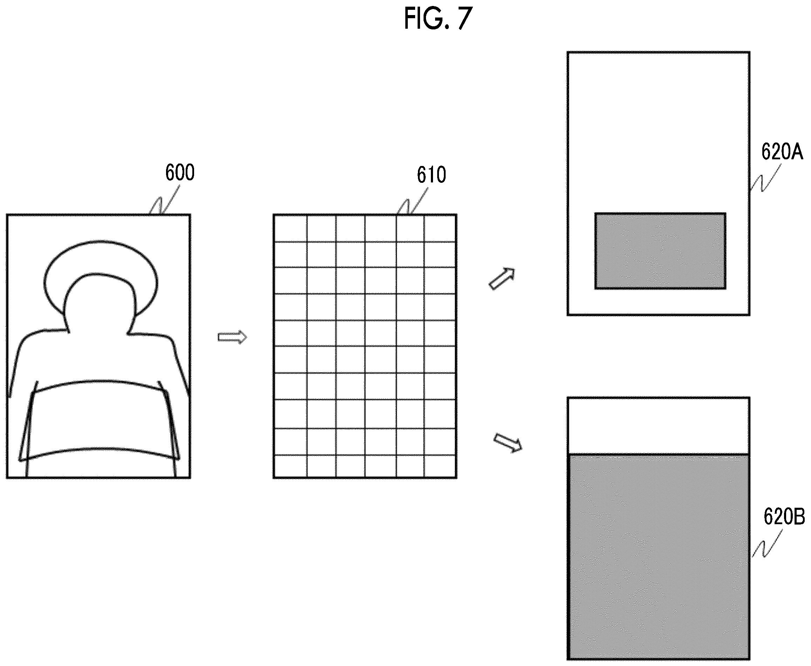

An example of the mask for extracting two regions is shown in Fig. 7. Fig. 7 shows an example in which two types of masks 620A and 620B are applied to a body movement map 610 calculated for each small region of a (resized) camera image 600. Among these, the mask 620A is used to extract the imaging region, and in a case where the imaging device 20 is an MRI device, a mask in which the region of the subject, on which the receive coil is mounted, is set to 1 and other regions are set to 0 can be used. The position where the receive coil is mounted may be determined from the image of the camera 33A or may be determined based on the center position of the examination space and the size of the receive coil because the subject is disposed such that the center position of the receive coil is located substantially near the center of the examination space in the MRI device.

Meanwhile, since the mask 620B is used to extract a region that includes the imaging region and that is wider than the imaging region, and a mask in which the region, in which the subject is present, is set to 1 and other regions are set to 0 can be used.

By monitoring the body movement of the region extracted with the mask 620A, it is possible to detect the body movement that directly affects the imaging, and it is possible to feed back the information to the imaging. In addition, by monitoring the body movement of the region extracted with the mask 620B, it is possible to detect a significant movement of the subject during the examination even in a case where the movement does not directly affect the imaging, and it is possible to achieve the watching function such as issuing an alert.

However, the region determined by the region determination section 11, that is, the mask generated by the mask generation section 12, is not limited to the above-described example, and one or a plurality of (two or more) regions may be set by taking into consideration the examination part, the ease of a subject movement (children or the like), or the like. For example, in a case of a receive coil in which a plurality of small coils are connected, a region where the receive coil is mounted may be divided into a plurality of regions, or may be divided into a region on a head part side including the receive coil, a region on a leg part side including the receive coil, and the like.

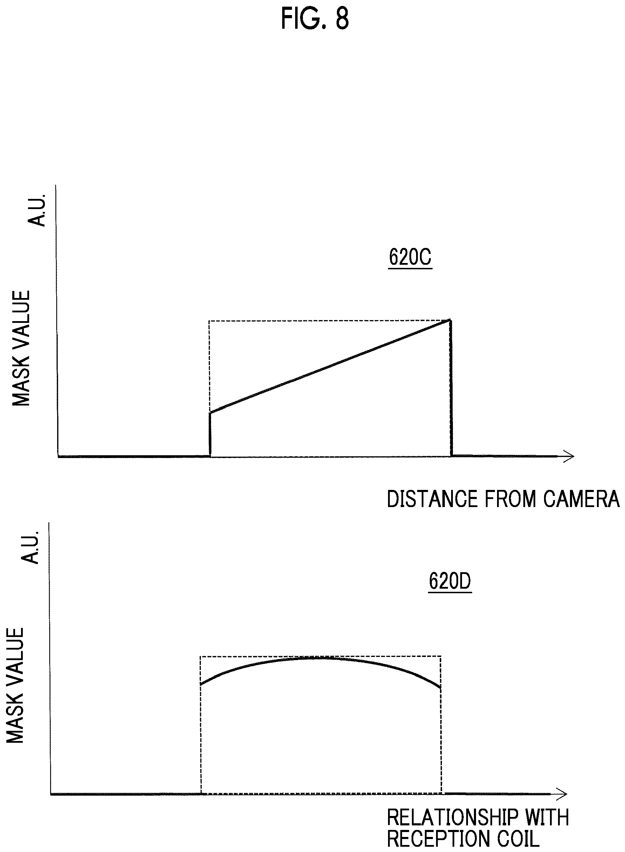

Further, the mask need not be a 1/0 binary mask, and may be weighted with a predetermined weight in consideration of a positional relationship with the camera, a sensitivity distribution of the receive coil, and the like. For example, as in a mask 620C shown on the upper side of Fig. 8, the farther the distance from the camera is, the larger the weight may be assigned because the body movement is captured smaller, and the closer the distance from the camera is, the smaller the weight may be assigned. Further, since the sensitivity of the receive coil is generally high at the center and it is considered that the influence of the body movement at that portion increases, the weight may be increased at the center, and the weight may be decreased in the periphery, as in a mask 620D shown on the lower side of Fig. 8.

In the above description, an example of a spatial mask has been described, but a temporal element and the magnitude of the body movement may further be added. The temporal element is, for example, a temporal mask indicating whether it is at the time of imaging or during the examination including before and after the imaging. One or a plurality of threshold values are set depending on whether or not the magnitude of the body movement affects the image and whether or not the magnitude of the body movement can be processed with the body movement correction. Regarding the threshold value of the magnitude of the body movement, the magnitude of the body movement that affects the image may be obtained in advance through imaging or simulation using a phantom.

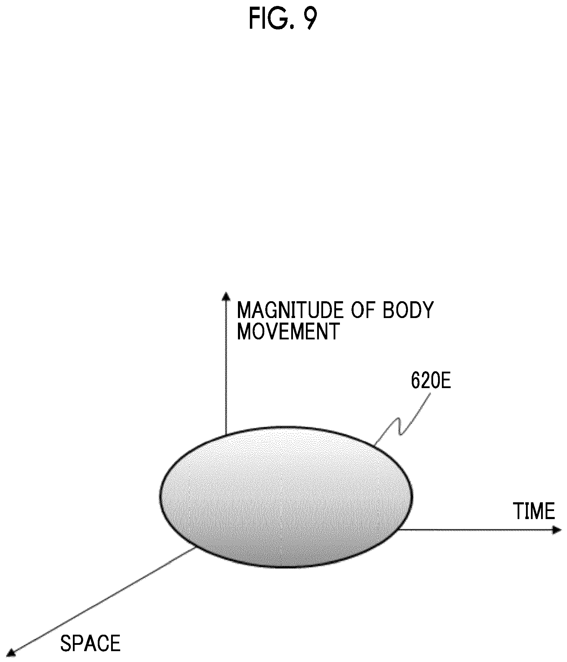

Fig. 9 shows a concept of a three-dimensional mask 620E in which the temporal element and the magnitude of the body movement are combined. By using such a three-dimensional mask, the body movement correction function and the watching function can be applied more appropriately. Specifically, by using a three-dimensional mask in which a mask for selecting a receive coil sensitivity distribution region as the spatial mask, a mask for selecting the time of imaging as the temporal mask, and a mask for selecting a range of equal to or greater than a first threshold value and equal to or less than a second threshold value as the magnitude of the body movement are combined, it is possible to utilize the body movement information extracted with the three-dimensional mask in the body movement correction function. In addition, by using a three-dimensional mask for selecting the entire region within the bore as the spatial mask, selecting "during the examination" as the temporal mask, and selecting "equal to or greater than the second threshold value" as the magnitude of the body movement, it is possible to utilize the body movement information extracted with this three-dimensional mask in the watching function.

Instead of the three-dimensional mask, there may be cases where a mask in which the spatial element and the temporal element are combined or a mask in which the spatial element and the magnitude of the body movement are combined is used.

### Processing of Body Movement Information Other Than Respiratory Movement

The body movement information processing device 10 (movement information presentation section 17) monitors the body movement for the region extracted with the above-described mask (S17).

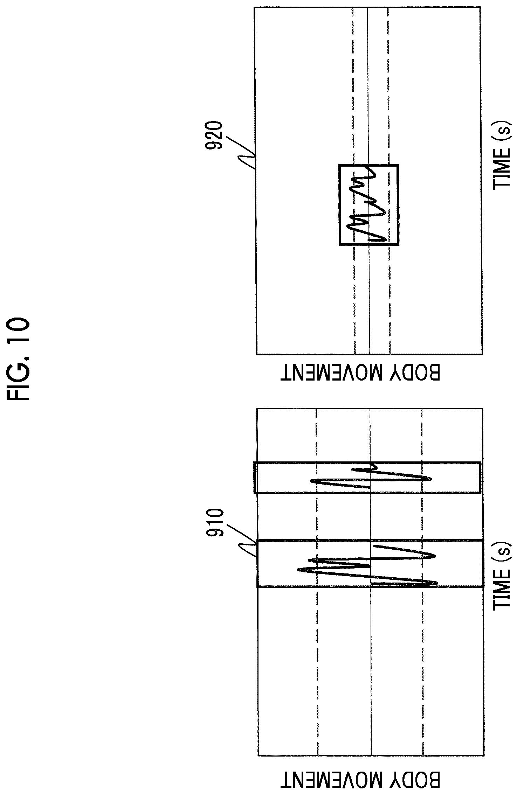

An example of the detected body movement information is shown in Fig. 10. As shown in the drawing, the body movement information is obtained in the form of a graph depicting variations in the magnitude of the body movement along the time axis (in arbitrary units). For example, in a graph 910 on the left side of Fig. 10, it can be seen that there were short-time movements with a large amplitude, for example, coughing or sneezing, and in a graph 920 on the right side of Fig. 10, it can be seen that there were small-amplitude movements lasting for a certain time or longer, for example, fidgeting.

The movement information presentation section 17 generates an alert based on the obtained body movement information. Therefore, the movement information presentation section 17 (monitoring window generation section 16) sets a window for conditions for issuing an alert regarding the body movement, determines whether the body movement corresponds to any of the conditions, and issues an alert. The window may be a window having only a threshold value of the magnitude of the body movement as shown in the graph of Fig. 10, but for example, three windows, that is, the amplitude of the body movement, the duration time of the body movement, and the time interval of the body movement, are set. The windows are set to indicate that the amplitude of the body movement is equal to or greater than a certain amplitude, indicate that the duration time of the body movement continues beyond a certain time, and determine a series of body movements based on the intervals at which the body movements are repeated after the time interval exceeds the threshold value of the amplitude and decreases once. Then, an alert is issued in a case where the body movement information calculated for the monitoring region falls within ranges determined by these three windows (S18). By setting the windows for the conditions including the magnitude and the time in this way, it is possible to obtain information not only about the magnitude of the movement but also about the characteristics thereof, and it is possible to issue an appropriate alert.

The setting of the windows for monitoring is not limited to the above example and is arbitrary. For example, an alert may be issued in a case where the amplitude of the body movement exceeds a predetermined threshold value regardless of the length of the duration time. Further, a window for watching function and a window for body movement correction may be prepared. In a case where the window for body movement correction is set, it is possible to reflect the fact that there is a body movement that, while not triggering an alert, still affects the imaging, and the time thereof in the operation of the imaging unit 21. For example, the imaging unit 21 can perform processing such as discarding measurement data of the time when the body movement has occurred, regaining the measurement data, and correcting the measurement data at that time based on the body movement information.

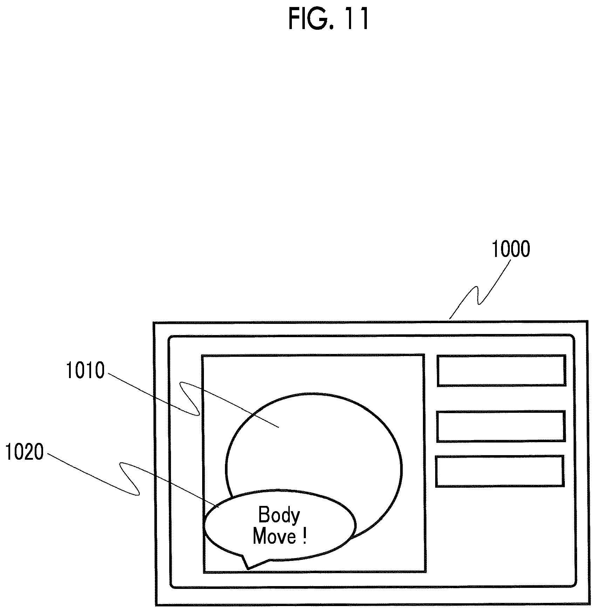

The method of issuing the alert is not particularly limited, and for example, the alert may be issued in the form of a speech bubble 1020 as shown in Fig. 11 on a display screen 1000 (for example, a screen on which a captured image 1010 is displayed) of the console 25 that the user is viewing, or a warning sound may be issued simultaneously or a voice alert may be utilized. In addition, these may be combined as appropriate. Further, the camera image at that time may be captured and displayed on the screen.

By issuing such an alert, it is possible to inform the user who is focused on other work that there has been an abnormal movement. In addition, by setting an appropriate window, it is possible to continue imaging as it is for movements that do not require an alert to be issued from the viewpoint of subject safety, such as in a case where the subject sneezes or coughs temporarily, and it is possible to avoid unnecessary interruptions in imaging.

### Processing of Respiratory Movement

In the watching function, in addition to non-steady body movements, steady respiratory movements are simultaneously detected and displayed for watching. In that case, processing for calculating the respiratory rate is further performed on the respiratory movements.

In this processing, first, the velocity vector Vresp is used to perform an integration in a time direction from a certain past time t1 to a current time t2 according to Equation (7), and a displacement x is calculated for each region (S24). $x = {\sum }_{t = t 1}^{t 2} {V}_{resp} \left(t\right)$

In determining a region that should be detected for the respiratory movements, for example, a region of the chest or abdomen is extracted using a mask. The mask is generated in advance by, for example, the mask generation section 12.

Regarding the method of determining the region that should be detected for the respiratory movements, the region that should be detected may be determined after calculating the respiratory movements for all the small regions within the camera image. For example, band power (BP) is calculated as a respiratory index (S25). BP is calculated by using Equation (8) to calculate a power spectral density P (f) for a frequency band that sufficiently includes respiratory components, for example, a frequency band of 0.1 to 0.5 Hz (equivalent to 6 to 30 breaths per minute when converted into the respiratory rate), and then calculating an average power within this frequency band as BP (Equation (8)). $P \left(f\right) = \frac{Δ t}{N} {\left|{\sum }_{n = 0}^{N - 1} {xe}^{- j 2 πf Δ tn}\right|}^{2}$ $BP = {\int }_{0.1}^{0.5} P \left(f\right) df$

In the equation, Δt is a sampling interval, and N is the number of sampling points.

The BP is calculated for all the small regions for which the velocity vectors are calculated, and for example, a region where BP is maximized is set as a respiratory detection ROI (S26). A respiratory waveform is obtained from displacement information (calculated in S24) obtained for the region set as the respiratory detection ROI, and the respiratory rate (per minute) is calculated (S27). The respiratory rate for one minute may be obtained by applying FFT to the respiratory waveform, calculating a peak frequency (Hz), and calculating 60 (sec)/peak frequency (Hz). In addition, the respiratory rate for one minute may be obtained from the time interval between the maximum value and the minimum value of the respiratory waveform. Further, a respiratory phase may be calculated together with the respiratory rate.

The movement information presentation section 17 sends the obtained respiratory rate to the imaging device 20 and displays the obtained respiratory rate on the display device (S28). The respiratory waveform may be displayed together with the respiratory rate. This information can be used not only for the user to understand the respiratory state of the subject but also for respiratory-synchronized imaging or the like by using the respiratory phase.

### Use of Body Movement Information S5

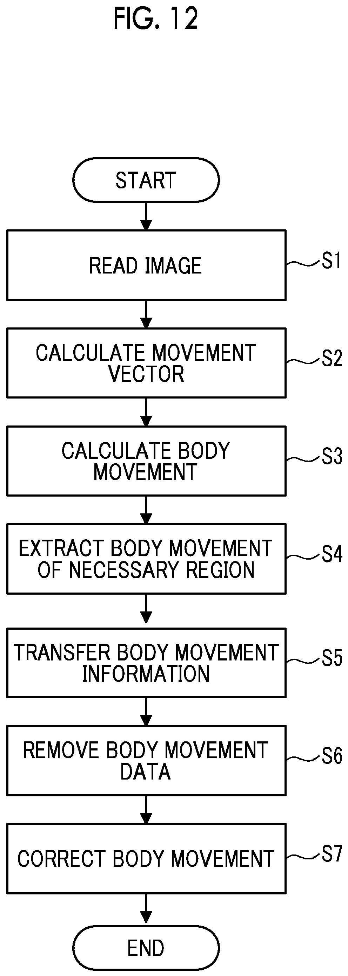

For example, as shown in Fig. 10, information indicating the time when the body movement has occurred and the magnitude of the body movement is sent to the imaging device 20, as the body movement information. The signal processing unit 23 of the imaging device 20 uses this body movement information to remove the k-space data collected at the time when the body movement has occurred, and performs the image reconstruction operations using the other k-space data. The outline is as follows. As shown in Fig. 12, as the removal method, for example, based on the waveform of a time history of the body movement, a time history mask for body movement removal is first created by setting a value equal to or greater than a certain threshold value to 1 and a value less than the threshold value to 0. After that, a k-space mask for body movement removal is created by taking into consideration the time history and the filling method of the k-space, whereby the k-space data collected at the time when the body movement has occurred is removed (S6).

A part of the k-space data is thinned out to perform image reconstruction, and the body movement is corrected (S7). As a technique for correcting the body movement, for example, parallel imaging reconstruction, sequential reconstruction in which iterative computational operations are performed while maintaining consistency of the measurement data, and the like have been developed, and these techniques can be used. In addition, in a case where there is a large amount of data at the time when the body movement has occurred, in a case where the data is low-frequency region data of the k-space by which image contrast is determined, or the like, the imaging unit 21 may be controlled and re-imaging may be performed to collect data when the body movement has occurred.

As described above, according to the present embodiment, by extracting and monitoring body movements of a predetermined region using a plurality of masks, it is possible to appropriately utilize the body movement information obtained from the extraction and monitoring for the body movement correction or the watching, which makes it possible to reduce unnecessary processing caused by over-detection.

Further, as the mask, by adding the magnitude of the body movement and the temporal element in addition to the spatial element, it is possible to allocate more appropriate functions.

In the above embodiment, in order to perform both the body movement correction function and the watching function, two body movement monitoring regions are set, and the body movements of the two regions are monitored using a mask corresponding to each region, but it is also possible to realize the body movement correction function and the watching function by setting only one region and using the body movement information extracted for that region. In addition, although a case where two functions are realized has been described in the present embodiment, the present invention also includes an imaging system that realizes only the watching function of monitoring body movements other than respiratory movements or only the body movement correction function.

Further, although the embodiment has been described using a case where the imaging device is an MRI device as an example, the present invention is not limited to the MRI device, and the present invention can be applied to various image diagnostic devices in which body movements are a problem, and the same effect can be obtained. For example, even in a case where the imaging device is an X-ray CT device, it is possible to measure the body movement of the subject, which has occurred during the rotation of a scanner, with a camera installed near the scanner, detect the body movement from the camera image, and extract the body movement of a predetermined region with a predetermined mask, and it is possible to understand at which point in time (angle) during the scanning the body movement that affects the examination or the imaging has occurred.

### Embodiment of MRI device

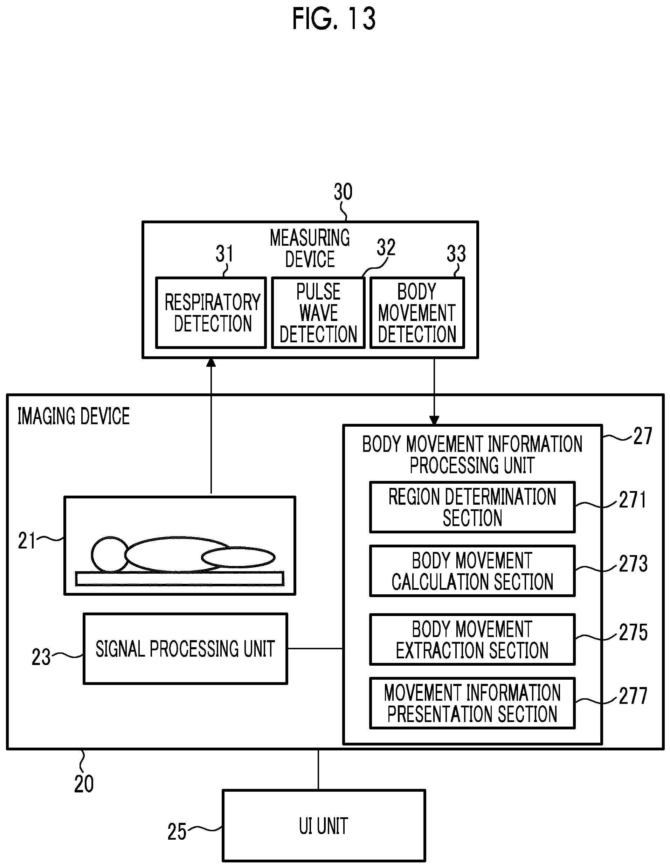

Next, an embodiment of the MRI device will be described. The MRI device of the present embodiment is characterized in that a known MRI device is provided with the functions of the body movement information processing device described above, and comprises, as shown in Fig. 13, the imaging unit 21, the signal processing unit 23, and a body movement information processing unit 270. As a device independent of the MRI device or an accessory device, the measuring device 30 that measures movement information of the subject disposed in the MRI device is installed. Similar to the above-described embodiment, the measuring device 30 is an instrument mounted on the subject disposed in the imaging unit 21, a device such as a pulse wave meter, a camera installed in the imaging unit (gantry), or the like, and the measuring device 30 itself may be provided with a body movement detection or body movement calculation function.

The configuration of the imaging unit 21 is the same as the configuration shown in Fig. 2, and duplicate descriptions of individual elements will be omitted. In the MRI device of the present embodiment, in addition to the signal processing unit 23 that performs various computational operations such as image reconstruction by using a nuclear magnetic resonance signal acquired by the imaging unit 21, the body movement information processing unit 270 that receives the signal from the measuring device 30 and collects the movement information of the subject is provided. A part or all of the functions of the signal processing unit 23 and the body movement information processing unit 270 can be realized by a computer (a computer provided with a CPU or a GPU, and a memory) incorporated into the MRI device.

Similar to the body movement information processing device 10 shown in Fig. 1 or 5, the body movement information processing unit 270 comprises a region determination section 271, a body movement calculation section 273, a body movement extraction section 275, and a movement information presentation section 277. Although the mask generation section and the monitoring window generation section shown in Fig. 5 are omitted in Fig. 12, these functional units may be provided.

The body movement information processing unit 270 acquires the body movement information detected by the measuring device 30 and performs processing necessary for the body movement correction and the watching and information presentation. Specifically, the processing as shown in Fig. 4, 6, or 12 is performed. A detailed description of the processing will be omitted, but the outline thereof is as follows. As shown in Fig. 4, an image is read from the measuring device (camera 33A) (S1), the body movement calculation section 273 calculates the movement vector and calculates the body movement (S2, S3). The region determination section 271 determines a region that should be monitored for the body movement and sets the mask, according to whether the purpose of the body movement calculation is the body movement correction or the watching function, and the body movement extraction section 275 uses the set mask to extract the region that should be monitored (S4).

The movement information presentation section 277 determines, regarding the body movement of a predetermined region extracted by the body movement extraction section 275, whether the body movement correction is necessary or an alert needs to be issued by using the threshold value of the magnitude or the duration time of the body movement, and transfers the obtained body movement information to the imaging unit 21 (S5) and presents the obtained body movement information as the alert as necessary (S6).

According to the present embodiment, by adding a function (body movement information processing) of processing information from the measuring device as a signal processing function of the MRI device, it is possible to use the detected body movement to perform prompt responses to the subject or reflection in imaging operations.

### Explanation of References

10: body movement information processing device
11: region determination section
12: mask generation section
13: body movement calculation section
15: body movement extraction section
17: movement information presentation section
20: imaging device
21: imaging unit
23: signal processing unit
25: console (display device)
30: measuring device
31: respiratory movement monitor
32: heart rate monitor
33: body movement monitor
33A: camera
270: body movement information processing unit
271: region determination section
273: body movement calculation section
275: body movement extraction section
277: movement information presentation section

## Claims

1. A body movement information processing device configured to receive a signal from a measuring device (30) which is configured to measure movement information of a subject disposed in an imaging device (20) and that is configured to process the movement information of the subject, the body movement information processing device comprising:
a body movement calculation section (13, 273) configured to calculate a body movement of the subject by using the signal from the measuring device;
a region determination section (11, 271) configured to determine a spatial region of a movement of the subject; and
a body movement extraction section (15, 275) configured to extract the body movement calculated by the body movement calculation section for the region determined by the region determination section,
**characterized by**
a mask generation section (12) configured to generate a mask (620) based on the spatial region determined by the region determination section (11, 271),
wherein the body movement extraction section (15, 275) is configured to extract the body movement by using the mask (620), and
wherein the mask (620) is a mask (620E, 620D) including weighting corresponding to at least one of a distance between the measuring device (30) and each position of the spatial region or a measurement sensitivity distribution of the imaging device (20).

2. The body movement information processing device according to claim 1,
wherein the region determination section (11, 271) is configured to determine a region that is a part of the subject and that includes an imaging target part, as the spatial region. or
wherein the region determination section (11, 271) is configured to determine a region that includes an imaging target part of the subject and a region other than the imaging target part, as the spatial region, or
wherein the region determination section (11, 271) is configured to determine a first region that is a part of the subject and that includes an imaging target part, and a second region that includes the imaging target part of the subject and a region other than the imaging target part, as the spatial regions, respectively.

3. The body movement information processing device according to claim 1 or 2,
wherein the mask includes a temporal range in which the body movement is extracted by the body movement extraction section (15, 275), in addition to the spatial region, and/or
wherein the mask includes a range of a magnitude of the body movement calculated by the body movement calculation section (15, 275), in addition to the spatial region.

4. The body movement information processing device according to any of claims 1 to 3,
wherein the body movement calculation section (13, 273) is configured to calculate movement vectors in two directions orthogonal to each other and use the movement vectors in the two directions to discriminate between a periodic movement and a non-steady movement orthogonal to the periodic movement, for calculation.

5. The body movement information processing device according to claim 4,
wherein the body movement extraction section (15, 275) is configured to set a threshold value for at least one of a magnitude, a duration time, or an occurrence interval with respect to the body movement calculated by the body movement calculation section (13, 273) and extract the non-steady movement of the subject based on the threshold value.

6. The body movement information processing device according to claim 5, further comprising:
a movement information presentation section (17, 277) configured to present information regarding the non-steady movement extracted by the body movement extraction section (15, 275) to an outside.

7. The body movement information processing device according to claim 4,
wherein the body movement calculation section (13, 273) is configured to calculate at least one of a displacement, a period, or the number of times per predetermined time for the periodic movement.

8. A magnetic resonance imaging device comprising:
an imaging unit (21) configured to measure a nuclear magnetic resonance signal generated by a subject and acquire an image of the subject; and
a body movement information processing unit (270) configured to receive a signal from a measuring device (30) that is configured to measure information regarding a movement of the subject, and collect movement information of the subject,
wherein the body movement information processing unit (270) includes:
a body movement calculation section (273) configured to calculate a body movement of the subject by using the signal from the measuring device,
a region determination section (271) configured to determine a spatial region of the movement of the subject,
a body movement extraction section (275) configured to extract the body movement calculated by the body movement calculation section for the region determined by the region determination section, and
a mask generation section (12) configured to generate a mask (620) based on the spatial region determined by the region determination section (11, 271),
wherein the body movement extraction section (275) is configured to extract the body movement by using the mask (620), and
wherein the mask (620) is a mask (620E, 620D) including weighting corresponding to at least one of a distance between the measuring device (30) and each position of the spatial region or a measurement sensitivity distribution of the imaging device (20).

9. The magnetic resonance imaging device according to claim 8,
wherein the imaging unit (21) is configured to, based on information on a non-steady movement extracted by the body movement extraction section (275), delete measurement data obtained in a case where the movement has occurred, and perform image reconstruction by using measurement data other than the deleted measurement data.

10. The magnetic resonance imaging device according to claim 8 or 9,
wherein the imaging unit (21) is configured to output an alert based on information on a non-steady movement extracted by the body movement extraction section (275).

11. The magnetic resonance imaging device according to any of claims 8 to 10,
wherein the region determination section (271) is configured to determine a region of the subject where a receive coil of the magnetic resonance imaging device is disposed, as the spatial region, or
wherein the region determination section (271) is configured to determine a region of the subject located within an imaging space of the magnetic resonance imaging device, as the spatial region.

## Patentansprüche

1. Körperbewegungs-Informationsverarbeitungsvorrichtung, die so konfiguriert ist, dass sie ein Signal von einer Messvorrichtung (30) empfängt, die so konfiguriert ist, dass sie Bewegungsinformationen einer Untersuchungsperson misst, die in einer Bildgebungsvorrichtung (20) angeordnet ist, und die so konfiguriert ist, dass sie die Bewegungsinformationen der Untersuchungsperson verarbeitet, wobei die Körperbewegungs-Informationsverarbeitungsvorrichtung umfasst:
einen Körperbewegungs-Berechnungsabschnitt (13, 273), der so konfiguriert ist, dass er eine Körperbewegung der Untersuchungsperson durch Verwenden des Signals von der Messvorrichtung berechnet;
einen Bereichsbestimmungsabschnitt (11, 271), der so konfiguriert ist, dass er einen räumlichen Bereich einer Bewegung der Untersuchungsperson bestimmt; und
einen Körperbewegungs-Extraktionsabschnitt (15, 275), der so konfiguriert ist, dass er die von dem Körperbewegungs-Berechnungsabschnitt berechnete Körperbewegung für den von dem Bereichsbestimmungsabschnitt bestimmten Bereich extrahiert,
**gekennzeichnet durch**
einen Maskenerzeugungsabschnitt (12), der so konfiguriert ist, dass er eine Maske (620) auf der Grundlage des von dem Bereichsbestimmungsabschnitt (11, 271) bestimmten räumlichen Bereichs erzeugt,
wobei der Körperbewegungs-Extraktionsabschnitt (15, 275) so konfiguriert ist, dass er die Körperbewegung durch Verwenden der Maske (620) extrahiert, und
wobei die Maske (620) eine Maske (620E, 620D) ist, die Gewichtung enthält, die mindestens einem von einem Abstand zwischen der Messvorrichtung (30) und jeder Position des räumlichen Bereichs und einer Messempfindlichkeitsverteilung der Bildgebungsvorrichtung (20) entspricht.

2. Körperbewegungs-Informationsverarbeitungsvorrichtung nach Anspruch 1,
wobei der Bereichsbestimmungsabschnitt (11, 271) so konfiguriert ist, dass er einen Bereich, der ein Teil der Untersuchungsperson ist und der einen Bildgebungszielteil enthält, als den räumlichen Bereich bestimmt, oder
wobei der Bereichsbestimmungsabschnitt (11, 271) so konfiguriert ist, dass er einen Bereich, der einen Bildgebungszielteil der Untersuchungsperson und einen anderen Bereich als den Bildgebungszielteil enthält, als den räumlichen Bereich bestimmt, oder
wobei der Bereichsbestimmungsabschnitt (11, 271) so konfiguriert ist, dass er einen ersten Bereich, der ein Teil der Untersuchungsperson ist und der einen Bildgebungszielteil enthält, und einen zweiten Bereich, der den Bildgebungszielteil der Untersuchungsperson und einen anderen Bereich als den Bildgebungszielteil enthält, entsprechend als die räumlichen Bereiche bestimmt.

3. Körperbewegungs-Informationsverarbeitungsvorrichtung nach Anspruch 1 oder 2,
wobei die Maske zusätzlich zu dem räumlichen Bereich einen zeitlichen Bereich enthält, in dem die Körperbewegung durch den Körperbewegungs-Extraktionsabschnitt (15, 275) extrahiert wird, und/oder
wobei die Maske zusätzlich zu dem räumlichen Bereich einen Bereich einer Größe der Körperbewegung, der durch den Körperbewegungs-Berechnungsabschnitt (15, 275) berechnet wird, enthält.

4. Körperbewegungs-Informationsverarbeitungsvorrichtung nach einem der Ansprüche 1 bis 3,
wobei der Körperbewegungs-Berechnungsabschnitt (13, 273) so konfiguriert ist, dass er zur Berechnung Bewegungsvektoren in zwei zueinander orthogonalen Richtungen berechnet und die Bewegungsvektoren in den zwei Richtungen verwendet, um zwischen einer periodischen Bewegung und einer nicht gleichmäßigen Bewegung orthogonal zu der periodischen Bewegung zu diskriminieren.

5. Körperbewegungs-Informationsverarbeitungsvorrichtung nach Anspruch 4,
wobei der Körperbewegungs-Extraktionsabschnitt (15, 275) so konfiguriert ist, dass er einen Schwellenwert für mindestens eines von einer Größe, einer Dauerzeit und einem Auftrittsintervall in Bezug auf die von dem Körperbewegungs-Berechnungsabschnitt (13, 273) berechnete Körperbewegung einstellt und die nicht gleichmäßige Bewegung der Untersuchungsperson auf der Grundlage des Schwellenwerts extrahiert.

6. Körperbewegungs-Informationsverarbeitungsvorrichtung nach Anspruch 5, ferner umfassend:
einen Bewegungs-Informationspräsentationsabschnitt (17, 277), der so konfiguriert ist, dass er Informationen in Bezug auf die nicht gleichmäßige Bewegung, die durch den Körperbewegungs-Extraktionsabschnitt (15, 275) extrahiert worden ist, nach außen präsentiert.

7. Körperbewegungs-Informationsverarbeitungsvorrichtung nach Anspruch 4,
wobei der Körperbewegungs-Berechnungsabschnitt (13, 273) so konfiguriert ist, dass er mindestens eine von einer Verschiebung, einer Periode und der Anzahl an Malen pro vorbestimmter Zeit für die periodische Bewegung berechnet.

8. Kernspintomographie-Vorrichtung, umfassend:
eine Bildgebungseinheit (21), die so konfiguriert ist, dass sie ein Kernmagnetresonanz-Signal, das von einer Untersuchungsperson erzeugt wird, misst und ein Bild der Untersuchungsperson erfasst; und
eine Körperbewegungs-Informationsverarbeitungseinheit (270), die so konfiguriert ist, dass sie ein Signal von einer Messvorrichtung (30) empfängt, die so konfiguriert ist, dass sie Informationen in Bezug auf eine Bewegung der Untersuchungsperson misst, und Bewegungsinformationen der Untersuchungsperson sammelt,
wobei die Körperbewegungs-Informationsverarbeitungseinheit (270) enthält:
einen Körperbewegungs-Berechnungsabschnitt (273), der so konfiguriert ist, dass er eine Körperbewegung der Untersuchungsperson durch Verwenden des Signals von der Messvorrichtung berechnet,
einen Bereichsbestimmungsabschnitt (271), der so konfiguriert ist, dass er einen räumlichen Bereich der Bewegung der Untersuchungsperson bestimmt,
einen Körperbewegungs-Extraktionsabschnitt (275), der so konfiguriert ist, dass er die von dem Körperbewegungs-Berechnungsabschnitt berechnete Körperbewegung für den von dem Bereichsbestimmungsabschnitt bestimmten Bereich extrahiert, und
einen Maskenerzeugungsabschnitt (12), der so konfiguriert ist, dass er eine Maske (620) auf der Grundlage des von dem Bereichsbestimmungsabschnitt (11, 271) bestimmten räumlichen Bereichs erzeugt,
wobei der Körperbewegungs-Extraktionsabschnitt (275) so konfiguriert ist, dass er die Körperbewegung durch Verwenden der Maske (620) extrahiert, und
wobei die Maske (620) eine Maske (620E, 620D) ist, die Gewichtung enthält, die mindestens einem von einem Abstand zwischen der Messvorrichtung (30) und jeder Position des räumlichen Bereichs und einer Messempfindlichkeitsverteilung der Bildgebungsvorrichtung (20) entspricht.

9. Kernspintomographie-Vorrichtung nach Anspruch 8,
wobei die Bildgebungseinheit (21) so konfiguriert ist, dass sie auf der Grundlage von Informationen über eine nicht gleichmäßige Bewegung, die durch den Körperbewegungs-Extraktionsabschnitt (275) extrahiert worden ist, Messdaten, die in einem Fall erhalten worden sind, in dem die Bewegung aufgetreten ist, löscht und Bildrekonstruktion durch Verwenden anderer Messdaten als der gelöschten Messdaten durchführt.

10. Kernspintomographie-Vorrichtung nach Anspruch 8 oder 9,
wobei die Bildgebungseinheit (21) so konfiguriert ist, dass sie eine Warnung auf der Grundlage von Informationen über eine nicht gleichmäßige Bewegung, die durch den Körperbewegungs-Extraktionsabschnitt (275) extrahiert worden ist, ausgibt.

11. Kernspintomographie-Vorrichtung nach einem der Ansprüche 8 bis 10,
wobei der Bereichsbestimmungsabschnitt (271) so konfiguriert ist, dass er einen Bereich der Untersuchungsperson, in dem eine Empfangsspule der Kernspintomographie-Vorrichtung angeordnet ist, als den räumlichen Bereich bestimmt, oder
wobei der Bereichsbestimmungsabschnitt (271) so konfiguriert ist, dass er einen Bereich der Untersuchungsperson, der sich innerhalb eines Bildgebungsraums der Kernspintomographie-Vorrichtung befindet, als den räumlichen Bereich bestimmt.

## Revendications

1. Dispositif de traitement d'informations de mouvement du corps configuré pour recevoir un signal provenant d'un dispositif de mesure (30) qui est configuré pour mesurer des informations de mouvement d'un sujet disposé dans un dispositif d'imagerie (20) et configuré pour traiter les informations de mouvement du sujet, le dispositif de traitement d'informations de mouvement du corps comprenant :
une section de calcul de mouvement du corps (13, 273) configurée pour calculer un mouvement du corps du sujet à l'aide du signal du dispositif de mesure ;
une section de détermination de région (11, 271) configurée pour déterminer une région spatiale d'un mouvement du sujet ; et
une section d'extraction de mouvement du corps (15, 275) configurée pour extraire le mouvement du corps calculé par la section de calcul de mouvement du corps pour la région déterminée par la section de détermination de région,
**caractérisé par**
une section de génération de masque (12) configurée pour générer un masque (620) sur la base de la région spatiale déterminée par la section de détermination de région (11, 271),
dans lequel la section d'extraction de mouvement du corps (15, 275) est configurée pour extraire le mouvement du corps à l'aide du masque (620), et
dans lequel le masque (620) est un masque (620E, 620D) incluant une pondération correspondant à au moins l'une d'une distance entre le dispositif de mesure (30) et chaque position de la région spatiale ou d'une distribution de sensibilité de mesure du dispositif d'imagerie (20).

2. Dispositif de traitement d'informations de mouvement du corps selon la revendication 1,
dans lequel la section de détermination de région (11, 271) est configurée pour déterminer, comme région spatiale, une région qui est une partie du sujet et qui inclut une partie cible d'imagerie, ou
dans lequel la section de détermination de région (11, 271) est configurée pour déterminer, comme région spatiale, une région qui inclut une partie cible d'imagerie du sujet et une région autre que la partie cible d'imagerie, ou
dans lequel la section de détermination de région (11, 271) est configurée pour déterminer, comme régions spatiales respectivement, une première région qui est une partie du sujet et qui inclut une partie cible d'imagerie, et une deuxième région qui inclut la partie cible d'imagerie du sujet et une région autre que la partie cible d'imagerie.

3. Dispositif de traitement d'informations de mouvement du corps selon la revendication 1 ou la revendication 2,
dans lequel le masque inclut une plage temporelle dans laquelle le mouvement du corps est extrait par la section d'extraction de mouvement du corps (15, 275), en plus de la région spatiale, et/ou
dans lequel le masque inclut une plage d'une amplitude du mouvement du corps calculé par la section de calcul de mouvement du corps (15, 275), en plus de la région spatiale.

4. Dispositif de traitement d'informations de mouvement du corps selon l'une quelconque des revendications 1 à 3,
dans lequel la section de calcul de mouvement du corps (13, 273) est configurée pour calculer des vecteurs de mouvement dans deux directions orthogonales l'une à l'autre et utiliser les vecteurs de mouvement dans les deux directions pour discriminer entre un mouvement périodique et un mouvement non stationnaire orthogonal au mouvement périodique, pour le calcul.

5. Dispositif de traitement d'informations de mouvement du corps selon la revendication 4,
dans lequel la section d'extraction de mouvement du corps (15, 275) est configurée pour définir une valeur seuil pour au moins l'un d'une amplitude, d'une durée ou d'un intervalle d'occurrence par rapport au mouvement du corps calculé par la section de calcul de mouvement du corps (13, 273) et extraire le mouvement non stationnaire du sujet sur la base de la valeur seuil.

6. Dispositif de traitement d'informations de mouvement du corps selon la revendication 5, comprenant en outre :
une section de présentation d'informations de mouvement (17, 277) configurée pour présenter à l'extérieur des informations concernant le mouvement non stationnaire extrait par la section d'extraction de mouvement du corps (15, 275).

7. Dispositif de traitement d'informations de mouvement du corps selon la revendication 4,
dans lequel la section de calcul de mouvement du corps (13, 273) est configurée pour calculer au moins l'un d'un déplacement, d'une période ou du nombre de fois par durée prédéterminée pour le mouvement périodique.

8. Dispositif d'imagerie par résonance magnétique comprenant :
une unité d'imagerie (21) configurée pour mesurer un signal de résonance magnétique nucléaire généré par un sujet et acquérir une image du sujet ; et
une unité de traitement d'informations de mouvement du corps (270) configurée pour recevoir un signal provenant d'un dispositif de mesure (30) qui est configuré pour mesurer des informations concernant un mouvement du sujet, et collecter des informations de mouvement du sujet,
dans lequel l'unité de traitement d'informations de mouvement du corps (270) inclut :
une section de calcul de mouvement du corps (273) configurée pour calculer un mouvement du corps du sujet à l'aide du signal provenant du dispositif de mesure,
une section de détermination de région (271) configurée pour déterminer une région spatiale du mouvement du sujet,
une section d'extraction de mouvement du corps (275) configurée pour extraire le mouvement du corps calculé par la section de calcul de mouvement du corps pour la région déterminée par la section de détermination de région, et
une section de génération de masque (12) configurée pour générer un masque (620) sur la base de la région spatiale déterminée par la section de détermination de région (11, 271),
dans lequel la section d'extraction de mouvement du corps (275) est configurée pour extraire le mouvement du corps à l'aide du masque (620), et
dans lequel le masque (620) est un masque (620E, 620D) incluant une pondération correspondant à au moins l'une d'une distance entre le dispositif de mesure (30) et chaque position de la région spatiale ou d'une distribution de sensibilité de mesure du dispositif d'imagerie (20).

9. Dispositif d'imagerie par résonance magnétique selon la revendication 8,
dans lequel l'unité d'imagerie (21) est configurée pour, sur la base d'informations sur un mouvement non stationnaire extraites par la section d'extraction de mouvement du corps (275), supprimer des données de mesure obtenues dans un cas où le mouvement s'est produit, et effectuer une reconstruction d'image à l'aide de données de mesure autres que les données de mesure supprimées.

10. Dispositif d'imagerie par résonance magnétique selon la revendication 8 ou la revendication 9,
dans lequel l'unité d'imagerie (21) est configurée pour émettre une alerte sur la base d'informations sur un mouvement non stationnaire extraites par la section d'extraction de mouvement du corps (275).

11. Dispositif d'imagerie par résonance magnétique selon l'une quelconque des revendications 8 à 10,
dans lequel la section de détermination de région (271) est configurée pour déterminer, comme région spatiale, une région du sujet où une bobine de réception du dispositif d'imagerie par résonance magnétique est disposée, ou
dans lequel la section de détermination de région (271) est configurée pour déterminer, comme région spatiale, une région du sujet située dans un espace d'imagerie du dispositif d'imagerie par résonance magnétique.
